# EUROPEAN PATENT SPECIFICATION

(11) **EP 2 029 630 B1**
(45) Date of publication and mention of the grant of the patent: **26.01.2011**
(21) Application number: 07796205.8
(22) Date of filing: 14.06.2007
(51) Int. Cl.: C08F 2/48, C08F 8/50, A61K 6/08, A61K 6/09, C08G 18/32, C08G 18/67, C08G 18/73, C08G 18/81, C08L 75/16

(54) **LOW STRESS DENTAL COMPOSITE CONTAINING PHOTOPOLYMERIZABLE AND PHOTOCLEAVABLE RESIN**
DENTALKOMPOSIT MIT GERINGER SPANNUNG ENTHALTEND LICHTPOLYMERISIERBARES UND LICHTSPALTBARES HARZ
COMPOSITE DENTAIRE À FAIBLE CONTRAINTE COMPRENANT RÉSINE PHOTOPOLYMÉRISABLE ET PHOTOCLIVABLE

(30) Priority: 16.06.2006 US 814594 P
(43) Date of publication of application: 04.03.2009
(73) Proprietor: DENTSPLY International Inc., York, PA 17405-0872 (US)
(72) Inventor: JIN, Xiaoming, Middletown, DE 19709 (US); HAMMESFAHR, Paul, D., Wyoming, DE 19934 (US)
(74) Representative: Hartz, Nikolai
(86) International application number: PCT/US2007/014158
(87) International publication number: WO 2008/005173

(56) References cited:
- EP-A- 0 373 662
- EP-A- 1 422 563
- WO-A-95/10552
- DE-A1-102006 047 863
- JP-A- 10 226 157
- US-A1- 2005 182 148
- US-A1- 2005 197 422
- DATABASE CA [Online] CHEMICAL ABSTRACTS SERVICE, COLUMBUS, OHIO, US; 27 November 1993 (1993-11-27), KIMURA, TETSUYA ET AL: "UV-curable oligomers and their compositions" XP002457956 retrieved from STN Database accession no. 1993:628166 -& JP 05 117361 A (HAYAKAWA RUBBER, JAPAN) 14 May 1993 (1993-05-14)

## Description

### Field of the invention

The present invention relates to a dental composite according to claim 1 containing a specific photopolymerizable and photocleavable main monomer. The photopolymerizable & photocleavable (P&P) resin monomers and resin composite compositions, provide unique balanced overall performance including very low polymerization shrinkage and very low shrinkage stress as well. The photoreactive moiety incorporated into such new resin's main frame enables to make the resin and/or the cured resin networks that are based upon such resin photocleavable. Thus the polymerization rate of free radical reaction for (meth)acrylate-based resin systems should be substantially reduced since it alters the network formation process and consequently allows the shrinkage stress getting relief significantly. In addition, it is expected that radically polymerizable resin systems containing such P&P resin would find wide range application in restorative dentistry where the dimensional stability and contraction stress within cured materials are critical to the total performance.

### Background of the Invention

US2005197422 discloses dental or periodontal cement compositions comprising the photosensitive initiator 2-hydroxy-1-[4-(hydroxyethoxy)phenyl]-2-methyl-1- propanone (Irgacure 2959) covalently attached to a base prepolymer. US2005182148 discloses photosensitive adhesive compositions for dental use that contain a bifunctional monomer with a photo-labile center. Highly cross-linked polymers have been studied widely as matrices for composites, foamed structures, structural adhesives, insulators for electronic packaging, etc. The densely cross-linked structures are the basis of superior mechanical properties such as high modulus, high fracture strength, and solvent resistance. However, these materials are irreversibly damaged by high stresses due to the formation and propagation of cracks. Polymerization stress is originated from polymerization shrinkage in combination with the limited chain mobility. Which eventually leads to contraction stress concentration and gradually such a trapped stress would released and caused microscopically the damage in certain weak zone like interfacial areas. Macroscopically it was reflected as debonding, cracking, et al. Similarly, the origin of contraction stress in current adhesive restorations is also attributed to the restrained shrinkage while a resin composite is curing, which is also highly dependent on the configuration of the restoration. Furthermore, non-homogeneous deformations during functional loading can damage the interface as well as the coherence of the material. Various approaches have been exploring by limiting the overall stress generation either from the restorative materials, or by minimizing a direct stress concentration at the restored interface. It included, for example, new resin, new resin chemistry, new filler, new curing process, new bonding agent, and even new procedure.

There has been tremendous attention paid on new resin matrix development that could offer low polymerization shrinkage and shrinkage stress. For example, various structure and geometry derivatives of (meth)acrylate-based resin systems; non-(meth)acrylates resin systems, non-radical-based resin system. In addition, for light curable, low shrink dental composites, not only new resin systems and new photoinitiators, new filler and filter's surface modification have also been extensively explored, such as filler with various particle size and size distribution, from nanometer to micrometer, different shape, irregular as milled or spherical as-made. It can also be different in composition like inorganic, organic, hybrid. Although an incremental improvement has been achieved with each approach and/or their mutual contribution, polymerization stress is still the biggest challenge in cured network systems.

This invention is related to a new kind of resin composition. However, unlike conventional resin system, a new concept is involved in designing such a new resin composition, which would render the polymerization stress in post-gel stage to a subsequent, selective network cleavage in order to have the stress partially released. As mentioned above, all of previous arts towards low shrink and low stress are based on the limitation on the shrink and stress formation in general. However, the shrinkage and stress development in cured network system should have two different stages: a pre-gel phase and a post-gel phase. Actually, most efforts of current arts are focussed on the pre-gel stage and some of them were proved effective. Unfortunately, these approaches become ineffective in terms to control the stress development in post-gel stage, where the shrinkage is not as much as in the pre-gel stage but the stress turns to much more sensitive to any polymerization extend. It is the immobility nature of the increasing crosslink density within the curing system that leads to the increasing stress concentration within the curing system, period. Even worse, the problem does not stop here and the trapped stress would eventually get relief from slow relaxation, which can create additional damage on a restored system. Therefore, our approach is based on such a concept that in the post-gel stage if some of "closed net" of any cross-linked system can be selectively broken to promote an extended stress relief period, the total stress concentration would be substantially reduced. To fulfil such a task, a photopolymerizable and photocleavable resin is proposed and a general molecular constitution is designed. It was expected that such a resin monomer can be polymerized like any other resin monomer but its mainframe is able to be triggered to break upon additional light source such as near UV is blended. This is a typical photocleavable process, but it is its capability to be photopolymerized and embedded into a cross-linked system make it unique. In addition, it also makes possible to avoid regenerating any leachable species through such secondary breakage.

Photocleavage is nothing new in solid synthesis of peptides, from which new peptides was directed on certain template in designed sequence, then it was cleaved from its template via a subsequent light exposure. There is no chemical contamination with such a process. On the other hand, photoacid and photobase could be viewed as extended applications for photocleavage. Acidic or basic component is temporally latent to avoid any unwanted interaction with others in the system and they can be released on demand such as light exposure to trigger the regeneration of the acid or base, which then act as normal acidic or basic catalyst for next step reactions. Recently, thermally removable or photo-chemically reversible materials are developed in order to make polymer or polymeric network depolymerizable or degradable for applications such as easily removing of fill-in polymer in MEMS, thermally labile adhesives, thermaspray coatings and removable encapsulation et al. Most recently, photocleavable dendrimers are explored in order to improve the efficiency for drug delivery. Based on our knowledge, there is no prior art involved photocleavale segment in cured network for contract stress control. However, all of those related arts could be used as a practical base to justify this investigation.

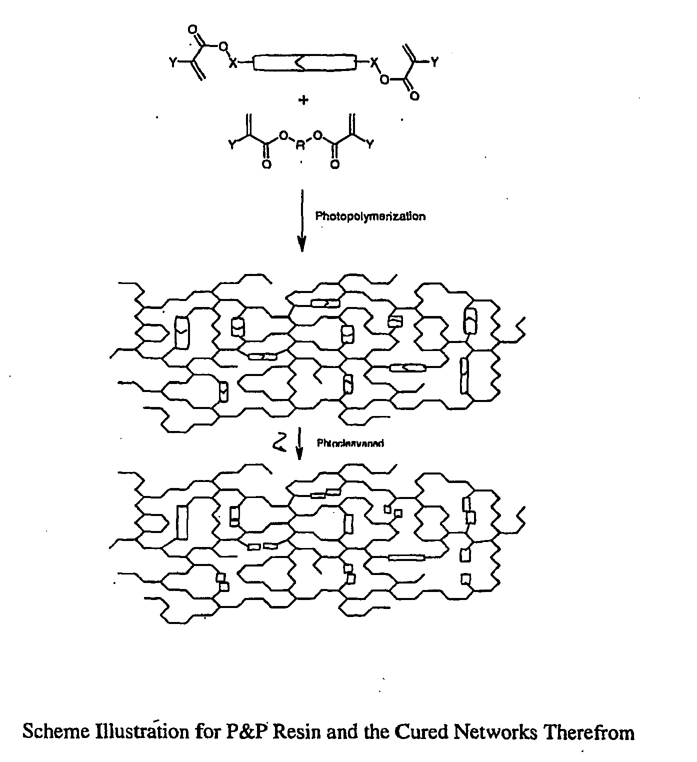

### APPROACH:

Theoretically speaking, if any somewhat environmentally sensitive moiety, such as a thermally cleavable or photo-labile linkage were incorporated into polymerizable resin monomers, such resin or its resulting polymeric material would become command-responsible, more specifically enable them thermo-cleavable or photo-cleavable. The chemistry of some classical photo-initiators could be adopted as the base for designing such photopolymerizable and photocleavable resin monomers, because such an initiator was explored as polymerizable photoinitiator or macroinitiator. However, none of them was really incorporated into polymer chain or polymeric network to make the polymeric chain or network breakable one way or another.

It is the another objective of this investigation to develop a new resin system for next generation low shrink and low stress restorative materials by incorporating a photocleavable or thermally liable moiety as part of a photopolymerizable resin monomer. It was expected with such an unusual approach it would enable a conventional polymerized network should be selectively cleavaged, thus to disperse the stress from postpolymerization and furthermore to result in a self stress-relief, ultimately to minimize the overall stress concentration.

In order to make a polymerized network cleavable-on-command by light or photocleavable, a light responsible moiety should be stable towards standard light exposure process such as visible light curing until additional exposure to specific light with distinguished energy level. In particular, such energy source can be anything other than the standard visible blue light. Near UV light would be one of typical examples among the many possible choices. Furthermore, it was expected that compounds derived from ortho-nitrobenzyl segment or from α-hydroxyalkylphenone should be ideal candidates for this new class resin monomers that be photopolymerized by visible light and be triggered to be breakable by extra UV light if needed.

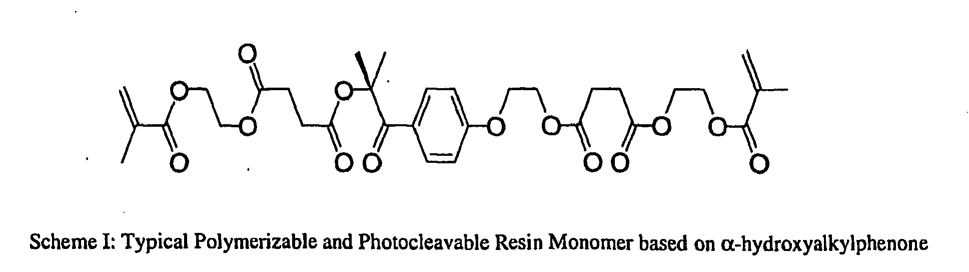

Its feasibility of this approach allows a rapid exploration on its versatility for a new class of resin. Accordingly, a variety of such polymerizable and photocleavable resin monomers was successfully prepared with wide range of constructions as illustrated in Scheme II.

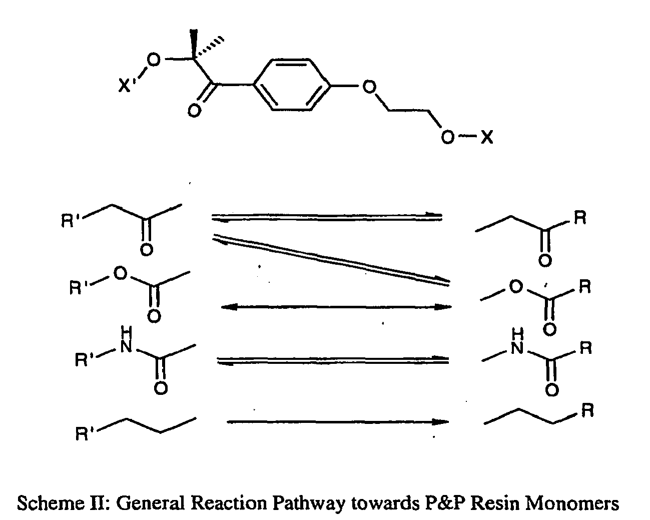

Thus, one of such α-hydroxyalkylphenone is 4-(2-hydroxyethoxy)-phenyl-2-hydroxy-2-methyl-2-propanone, HP, a classic α-hydroxyacetophenone photoinitiator. Its dual hydroxyl groups allow constructing a wide range of polymerizable monomers via various simple reactions as illustrated in Scheme II. For example HP can be incorporated with methacylate, acrylated or vinyl ether et al, via one or two-step reaction. Various photopolymerizable HP derivatives have been prepared via ester-ester, ester-carbonate, carbonate-carbonate, and urethane-urethane linkage. From them, the urethane-urethane linkage offers the best robust process: easy control, non-solvent process.

In addition, such new resin monomer was formulated with other conventional resin monomers like BisGMA, TEGDMA, UDMA or experimental resin monomer like macrocyclic resin in a variety ratio in order to have overall performance got balanced for the resulting composites. As showed in the following examples, remarkable low shrinkage, low stress and excellent mechanical property plus the good handling characteristics were demonstrated by those composites based on such new class P&P resin monomers.

As showed in the example, a typical urethane-based P&P resin can be easily prepared via a simple bulk process. Depending the nature of isocyanate, one or two-step reaction will be involved to prepare such a P&P resin. IEM is the simplest polymerizable isocyanate. Unfortunately, its toxicity limits its application as biomedical materials. Here we have successfully developed a simple two-step process to make such a photopolymerizable and photocleavable resin: in step I, HP was capped with diisocyanate to form new diisocyanate with well-controlled sequence, which was then further reacted with any (meth)acrylate containing a hydroxyl group as step II reaction. This procedure would not only allow carrying the reaction in one reactor, but also allow using that easily available dissocyanate instead of IBM.

Suitable diisocyanates include the alklene diisocyanates wherein the alkylene group ranges from 2 to about 18 carbon atoms and arylene and substituted arylene di-and polyisocyanates. Thus, exemplary diisoyanates and polyisocyanates include, for example:
Alklene diisocyanates
   ethylene diisocyanate
   propylene diisocyanate
   tetramethylene diisocyanate
   pentamethylene diisocyanate
   hexamethylene diisocyanate(HDI)
   hexamethylene diisocyanate biuret
   hexamethylene diisocyanate trimer(isocyanurate)
   octamethylene diisocyanate
   decamethylene diisocyanate
   undecamethylene diisocyanate
   dodecamethylene diisocyanate
   isophorone diisocyanate (IPDI)
   hydrogenated diphenyl methane diisocyanate (H₁₂MDI)
Arylene diisocyanate
   xylylene-1,4-diisocyanate(p-XDI)
   xylylene-1,3-diisocyanate(m-XDI)
   m-pheylene diisocyanate
   p-pheylene diisocyanate
   toluene-2,6-diisocyanate(2,6-TDI)
   toluene-2,4-diisocyanate(2,4-TDI)
   mesitylene diisocyanate
   durylene diisocyanate
   benzidene diisocyanate
   I-methyl phenylene-2,4-diisocyanate
   naphthylene-1,4-diisocyanate
   1,2,4-benzene triisocyanate
   4,4'-diisocyanato diphenyl methane(MDI)
   3,3'-dimethyl-4,4'-diisocyanato diphenyl methane
   4,4'-diphenyl propane diisocyanate
   dianisidine diisocyanate
   m-tetramethylenexylene diisocyanate(TMXDI)
In addition, suitable (meth)acrylate containing a hydroxyl group include
   2-hydroxyethyl (meth)acrylate
   2-hydroxypropyl (meth)acrylate
   3-(acryloxyl)-2-hydroxypropyl (meth)acrylate
   diethylene glycol monohydroxyl (meth)acrylate
   triethylene glycol monohydroxyl (meth)acrylate
   tetraethylene glycol monohydroxyl (meth)acrylate
   polyethylene glycol monohydroxyl (meth)acrylate

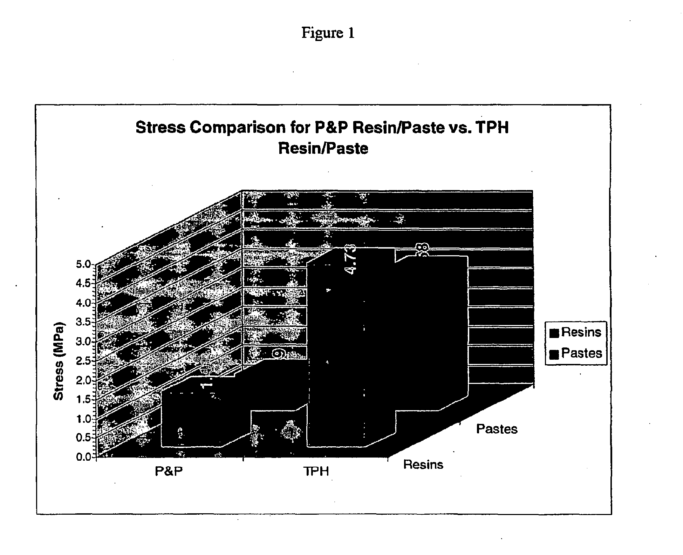

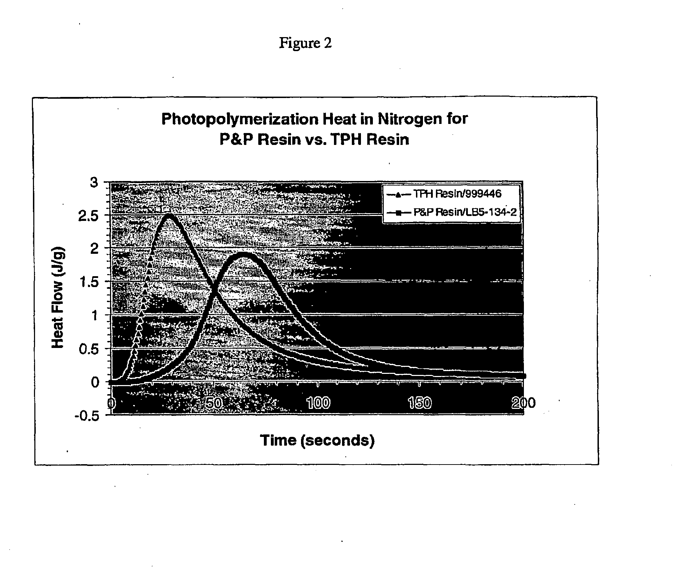

Surprisingly, it has now been found that the resinous dental compositions based on according to the invention for polymeric networks after curing thereof and even without addition of any filler, which has remarkably low polymerization stress even prior to a photocleavage (Figure 1). It is believed somehow the HP moiety incoporated in such novel P&P resin had involved in the radical reaction thus to slow polymerization rate during conventional photopolymerization while it was exposed to visible light, even though no cleavage occurred at this point. The relatively slow polymerization rate (Figure 2) for those P&P resin-based materials allow a substantial relaxation than those rapid cured systems.

**Table Ia: Polymerization Shrinkage and Stress for Various Activated Resin Mix**

| | *Shrinkage* (%) *by Helium Pycnometer* | *Stress (MPa) by Tensometer* |
|---|---|---|
| Denfortex Resin | 10.2 | 4.1 |
| TPH Resin/999446 | 6.8 | 4.5 |
| TPH Resin/999447 | 7.3 | 4.3 |
| Harpoon Resin/xj5-12 | 5.5 | 3.1 |
| Harpoon Resin/xj5-26 | 5.8 | 3.2 |
| | | |
| P&P Resin/LB5-158-1 | 5.2 | 1.4 |
| P&P Resin/LBS-167-3 | 6.2 | 1.5 |
| P&P Resin/L.BS-167-4 | 6.9 | 1.5 |
| P&P ResinALB6-54-1 | 5.6 | 0.6 |
| P&P Resin/LB6-54-2 | 5.5 | 0.7 |
| P&P Resin/LB6-58-4 | 6.2 | 1.2 |
| P&P Resin/LB6-73-1 | 6.3 | 1.7 |
| P&P Resin/LB6-73-2 | 5.9 | 1.6 |
| P&P Resin/LB5-114 | 6.0 | 1.4 |

**Table Ib: Polymerization Shrinkage and Stress for Various P&P/TPH Resin Mixtures**

| | *TPH Resin, 999446*/*030804* | *P&P Resin, LB6-114* | *Viscosity@20C,* | *Stress* | *Shrinkage* |
|---|---|---|---|---|---|
| | % | % | *Pa.s* | *MPa* | % |
| P&P Resin/LB6-114 | 0 | 100 | 102 | 1.41 | 5.96 |
| XJ5-208-1 | 10 | 90 | 74 | 1.92 | 6.17 |
| XJ5-208-2 | 20 | 80 | 61 | 2.17 | 6.37 |
| XJ5-208-3 | 30 | 70 | 47 | 2.51 | 6.54 |
| XJ5-208-4 | 40 | 60 | 39 | 2.75 | 6.70 |
| XJ5-208-5 | 50 | 50 | 32 | 2.81 | 6.76 |
| XJ5-208-6 | 60 | 40 | 27 | 3.22 | 6.90 |
| XJ5-208-7 | 70 | 30 | 24 | 3.69 | 7.00 |
| XJ5-208-8 | 80 | 20 | 21 | 3.99 | 7.10 |
| TPH Resin/999446 | 0 | 100 | 15 | 4.73 | 7.35 |

**Table II: Polymerization Shrinkage, Stress and Microstrain for Various Composites**

| | *Shrinkage* (%) *by Helium Pycnometer* | *Microstrain (ue) by Strain Gage* | *Stress (MPa) by Tensometer* |
|---|---|---|---|
| TPH/A2 | 3.10 | 1600 | 2.9 |
| EsthetX/A2 | 2.92 | 1995 | 2.5 |
| SureFil/A | 2.09 | 1840 | 2.7 |
| Supreme/A2B | 2.65 | 1720 | 2.8 |
| Supreme/YT | 2.39 | 2005 | 3.3 |
| Harpoon/A2 | 1.34 | 1000 | 1.7 |
| | | | |
| P&P/LB6-55 | 1.01 | N/A | 0.8 |
| P&P/LB6-56 | 1.07 | N/A | 0.8 |
| P&P/LB6-69 | 1.40 | N/A | 1.4 |
| P&P/LB6-74 | 1.68 | N/A | 1.3 |
| P&P/LB6-75 | 1.57 | N/A | 1.4 |
| P&P/LB6-115 | 1.82 | N/A | 1.5 |

**Table III: General Physical Property for Activated Neat P&P Resin**

| | *100% P&P Resin (LB6-72)* | *100% P&P Resin (EBR6983)* |
|---|---|---|
| | *0.15% CQ* | *0.15% CQ* |
| | *0.20% EDAB* | *0.20% EDAB* |
| | *0.02% BHT* | *0.02% BHT* |
| Lot # | LB6-73-2 | LB6-114 |
| Viscosity at 20°C poise | 1050 | 1020 |
| Uncured density g/cm³ | 1.1164 | 1.1162 |
| Cured density g/cm³ | 1.1865 | 1.1867 |
| Shrinkage @ 24hrs % | 5.91 | 5.96 |
| Stress @ 60min MPa | 1.6 | 1.4 |
| ΔH₁ in N2 w/o UV filter | 113 | 110 |
| to seconds | 20 | 15 |
| tₘₐₓ seconds | 38 | 31 |
| ΔH₁ in N2 w/ UV filter | 110 | 107 |
| to seconds | 23 | 17 |
| tₘₐₓ, seconds | 46 | 36 |

**Table IV: Physical and Mechanical Properties for Typical P&P Resin-based Composites**

| *Pastes* | *LB6-75* | *LB6-115* |
|---|---|---|
| Resin Composition | Lab Batch of P&P LB6-73-2 (18%) | P&P Resin (EBR6983) LB6-114 (18.5%) |
| Filler Composition | LB6-91-3 (82%) | LB6-91-3 (81.5%) |
| PZN Enthalpy ΔH(J/g) in N2 | (Vis/UV) 20/ | (Vis/UV) 21/ |
| Induction Time t₀ seconds | 22/ | 14/ |
| Peak Time tₘₐₓ seconds | 58/ | 44/ |
| Uncured density g/cm³ | 2.1553 | 2.1411 |
| Cured density g/cm³ | 2.1898 | 2.1808 |
| Shrinkage (%) by pycnometer @ 24hrs | 1.6 | 1.8 |
| Shrinkage Stress MPa | 1.4 | 1.6 |
| Flexural Strength MPa | 137+/-4 | 128+/-9 |
| Flexural Modulus MPa | 10800+/-440 | 9963+/-136 |
| Compressive Strength MPa | 344+/-11 | 316+/-24 |
| Compressive Modulus MPa | 8080+/-530 | 7920+/-214 |

The present invention will now be described in detail with reference to the following examples that by no means limit the scope of the invention.
The following abbreviations are used in the example.
BisGMA: 2,2-bis(4-(3-methacryloyloxy-2-hydroxypropoxy)-phenyl)propane
HP: 4-(2-hydroxyethoxy)-phenyl-2-hydroxy-2-metbyl-2-propanone
HEMA: 2-hydroxyethyl methacrylate
HEPA: 2-hydroxypropyl methacrylate
TEGDMA: triethylene glycol dimethacrylate
UDMA: di(methacryloxyethyl)trimethyl-1,6-hexaethylenediurethane
HEMASA: mono-2-(methacryl-oxy)ethyl-succinate
TMDI: 2,2,4(2,4,4)-trimethyl-1,6-hexanediisocyanate
HDI: hexamethylene diisocyanate
IEM: methacryloxyethyl isocyanate
ICEM: 1-methacryloxyethyl-2,4,4(2,2,4)-trimethyl-6-hexaneisocyanate
CDI: 1,1'-carbonyldiimidazole
BHT: butylhydroxytoluene
DBTDL:dibutyltin dilaurate
TCDCA: tricyclo[5.2.1.0^{2,6}] decane-dimethanol diacrylate

### Example 1

### Preparation of the adduct of IEM-HP-IEM (XJ5-129).

A 250ml three-necked flask equipped with a mechanical agitator, dry air inlet and water-cooling condenser, which was immersed in an oil-bath, was charged 25.5 grams of IEM, and 0.15gram of DBTDL and 0.1 gram of BHT. Then it was charged with 14.7 grams of grounded HP. The reaction was kept at 25°C with the oil-bath temperature, then the temperature was slowly raised to 30-35 °C for overnight reaction. The conversion of HP reached up to 95%. Additional diluent (33 grams of TCDCA) was used to adjust viscosity.

### Example 2

### Preparation of the adduct of HEMA-TMDI-HP-TMDI-HEMA (XJ5-140)

A 500ml three-necked flask equipped with a powder addition funnel, mechanical agitator, dry air inlet and water-cooling condenser, which was immersed in an oil-bath, was charged 71.5grams of TMDI, and 0.21 gram ofDBTDL. Then 22.4 grams of grounded HP were slowly in portion added into the flask in a period of 5-6hrs. In such a manner to avoid rapid reaction heat increase, which might jeopardize the desired sequence of TMDI-HP-TMDI. The fully conversion of HP as fully capped with TMDI can be easily determined by 1H NMR. Then 0.15gram of BHT was charged into the system. With continuous purge of dry air into the reaction system, 71.6 grams of HEMA was added into the flask through a dropping funnel during a period of 2hrs. An effective agitation is critical during the initial stage of HEMA addition in order to minimize the reaction rate so as to avoid overheat in the system, which can cause premature polymerization or gelation. The reaction temperature has to be controlled below 60 °C, best for below 45 °C. After HEMA addition, allow for additional 1hr reaction at 35-40 °C. Then additional diluent such as TEGDMA was charged into system and mixed for a couple of hours. Again 1H NMR was used to determine the HEMA/NCO conversion. A typical two-step reaction would be completed within14hrs with yield of 97-99%.

### Example 3

### Preparation of the adduct of HEMA-HDI-HP-HDI-HEMA (XJ5-136)

A 250ml three-necked flask equipped with a powder addition funnel, mechanical agitator, dry air inlet and water-cooling condenser, which was immersed in an oil-bath, was charged 40.8grams of HDI, and 0.17gram of DBTDL and 0.15 gram of BHT. Then 22.4 grams of grounded HP were slowly in portion added into the flask in a period of 3-4hrs. The reaction temperature was remained at 25 °C through the step. In such a manner to avoid rapid reaction heat increase, which might jeopardize the desired sequence of HDI-HP-HDI. The reaction was kept running overnight and the resulting resin turns opaque as evident of partially crystallized. The fully conversion of HP as fully capped with TMDI can be easily determined by 1H NMR. With continuous purge of dry air into the reaction system, 27.0 grams of HEMA was slowly added into the flask. The reaction temperature was raised to 35-40 °C. After additional 6hrs reaction at 35-40°C, 70 grams of diluent, was mixed with the resulting resin for a couple of hours prior to discharged.

### Example 4

### Preparation of the adduct of HEMA-TMDI-HP-TMDI-HEMA (LB6-73)

A 1000ml jacked, cylinder resin kettle equipped with a powder addition funnel, mechanical agitator, dry air inlet and water-cooling condenser, through which 35°C of heated water was circulated during the reaction, was charged 96.6 grams of TMDI, and 0.25gram of DBTDL. Then 35.5 grams of grounded HP were slowly in portion added into the flask in a period of 6hrs. The fully conversion of HP as fully capped with TMDI can be easily determined by 1H NMR. Then 0.20gram of BHT was charged into the system. With continuous purge of dry air into the reaction system, 86.2 grams of HEMA was added into the flask through a dropping funnel during a period of 2hrs. An effective agitation is critical during the initial stage of HEMA addition in order to minimize the reaction rate so as to avoid overheat in the system, which can cause premature polymerization or gelation. The reaction temperature has to be controlled below 60°C, best for below 45°C. After HEMA addition, allow for additional 1hr reaction at 35-40°C. Then additional diluent such as 30-40 grams of TEGDMA was charged into system and mixed for a couple of hours. Again 1H NMR was used to determine the HEMA/NCO conversion. A typical two-step reaction would be completed within14hrs with yield of 97-99%.

### Example 5 through 14

Per similar reaction procedure as described in example 4, Table I listed in details on other reaction runs at 35°C based on various compositions.

**Table I: Reaction Runs at 35°C with Different Compositions**

| *Resins* *Comps* | *TMDI* *g* | *HP* *g* | *D8TDL* *g* | *HEMA* *g* | *TEGDMA* *g* | *Re. Time* *hr* | *Yield* *%* | *Viscosity* *@20°C poise* | *Viscosity* *@50°C poise* |
|---|---|---|---|---|---|---|---|---|---|
| LB6-76 | 101.5 | 35.3 | 0.25 | 86.2 | 40 | 13 | 97 | 780 | 25 |
| LB6-78 | 91.9 | 35.1 | 0.25 | 86.2 | 40 | 12 | 97 | 340 | 20 |
| LB6-80 | 96.6 | 37.4 | 0.25 | 86.2 | 40 | 12 | 97 | 520 | 20 |
| LB6-81 | 96.7 | 33.3 | 0.25 | 86.2 | 40 | 12 | 98 | 460 | 20 |
| LN6-82 | 96.7 | 35.2 | 0.25 | 90.5 | 40 | 13 | 99 | 320 | 15 |
| LB6-83 | 96.7 | 35.2 | 0.25 | 81.4 | 40 | 12 | 98 | 710 | 25 |
| LB6-84 | 96.7 | 35.2 | 0.25 | 86.2 | 42 | 12 | 99 | 420 | 15 |
| LB6-85 | 96.7 | 35.2 | 0.25 | 86.2 | 38.5 | 13 | 98 | 530 | 15 |
| LB6-86 | 96.7 | 35.2 | 0.32 | 86.2 | 40 | 13 | 98 | 480 | 25 |
| LB6-87 | 96.7 | 35.6 | 0.20 | 86.2 | 30 | 14 | 98 | 950 | 35 |

### Example 15

### Preparation of the adduct of HP-HDI-HP (XJ5-160)

A 500ml three-necked flask equipped with a mechanical agitator, dry air inlet and water-cooling condenser, which was immersed in an oil-bath, was charged 9.7 grams of HDI, and 0.11gram of DBTDL and 19.3 grams of HP at room temperature. Then the reaction temperature was raised to 35°C and it was kept running overnight.

### Example 16

### Preparation of the adduct of HP-TMDI-HP (XJ5-169)

A 250ml three-necked flask equipped with a mechanical agitator, dry air inlet and water-cooling condenser, which was immersed in an oil-bath, was charged 250ml dry methylene dichloride, 22.45 grams of HP, and 0.14gram of DBTDL at room temperature. HP is insoluble in the solvent. Then 10.55 grams of grounded TMDI was added into the flask dropwisely within 1hr, then the reaction was kept running overnight. Then 50 grams of TEGDMA was mixed with the resulting viscose liquid.

### Example 17

### Preparation of the adduct of HP -TMDI-HEMA (XJ5-157)

A 250ml three-necked flask equipped with a mechanical agitator, dry air inlet and water-cooling condenser, which was immersed in an oil-bath, was charged 22.63 grams of TMDI, and 0.16gram of DBTDL at room temperature. Then 16.4 grams of HP in a dropping addition funnel was slowly added into the flask within 1.5hrs. After additional 1hr reaction, 18.8 grams of HEMA was charged slowly into the flask via the dropping addition funnel during a period of 2hrs. The reaction was kept running overnight.

### Comparative Example 1

### Preparation of the adduct of ICEM-HP-ICEM (XJ5-146)

A 500ml three-necked flask equipped with a mechanical agitator, dry air inlet and water-cooling condenser, which was immersed in an oil-bath, was charged 56.3grams of ICEM, and 20.0grams of TEGDMA. Then 0.11gram of DBTDL and 0.05gram of BHT. The bath temperature was set at 25°C. Then it was added all of 11.2 grams of grounded HP. The reaction occurred slowly as evident by the slow temperature increase. Heated up to 35°C and kept for additional 222hrs prior to reach its maximum conversion, 96%, as determined by same 1HNMR method as used above. Obviously as showed in Examples 3 through 14, the two-step reaction process is more efficient that one-step process as described in this control example.

## Claims

1. A dental composite containing a photopolymerizable and photocleavable resin monomer comprising a reactive photoresponsive α-hydroxyalkylphenone moiety and at least two photopolymerizable groups selected from the group consisting of vinyl, vinylether, acrylate and methacrylate groups.

2. The dental composite as in claim 1, wherein said α-hydroxyalkylphenone is 4-(2- hydroxyethoxy)-phenyl-2-hydroxy-2-methyl-2-propanone.

3. The dental composite as in claim 1, further comprising ester, carbonate, urea, urethane or ether linkages and combinations thereof.

4. The dental composite as in claim 1, formed as the derivative of a urethane-based monomer via a one-step or a two-step process.

5. The dental composite as in claim 4, wherein said urethane-based monomer is selected from the group consisting of alklene diisocyanates wherein said alkylene group has from 2 to 18 carbon atoms, and arylene and substituted arylene di-and poly-isocyanates.

6. The dental composite as in claim 5 wherein said cyanates are selected from the group consisting of ethylene diisocyanate, propylene diisocyanate, tetramethylene diisocyanate, pentamethylene diisocyanate, hexamethylene diisocyanate (HDI), hexamethylene diisocyanate biuret, hexamethylene diisocyanate trimer(isocyanurate), octamethylene diisocyanate, decamethylene diisocyanate, undecamethylene diisocyanate, dodecamethylene diisocyanate, isophorone diisocyanate (IPDI), hydrogenated diphenyl methane diisocyanate (Hi2MDI), xylylene-1,4-diisocyanate (p- XDI), xylylene-1,3-diisocyanate (m-XDI), m-pheylene diisocyanate, p-pheylene diisocyanate, toluene-2,6-diisocyanate (2,6-TDI), toluene-2,4-diisocyanate (2,4-TDI), mesitylene diisocyanate, durylene diisocyanate, benzidene diisocyanate, 1 -methyl phenylene-2,4-diisocyanate, naphthylene-1,4-diisocyanate, 1,2,4-benzene triisocyanate, 4,4'-diisocyanato diphenyl methane (MDI), 3,3'-dimethyl-4,4'- diisocyanato diphenyl methane, 4,4'-diphenyl propane diisocyanate, dianisidine diisocyanate, m-tetramethylenexylene diisocyanate (TMXDI). In addition, suitable (meth)acrylate containing a hydroxyl group include: 2-hydroxyethyl (meth)acrylate, 2-hydroxypropyl (meth)acrylate, 3-(acryloxyl)-2-hydroxypropyl (meth)acrylate, diethylene glycol monohydroxyl (meth)acrylate, triethylene glycol monohydroxyl (meth)acrylate, tetraethylene glycol monohydroxyl (meth)acrylate, polyethylene glycol monohydroxyl (meth)acrylate, and combinations thereof.

7. The dental composite as in claim 1, further comprising at least one additional resin selected from the group consiting of BisGMA, TEGDMA, UDMA and HEMA.

8. The dental composite as in claim 7, wherein the composite comprises monomer in an amount of from 1 to 99 percent by weight of the dental composite.

9. The dental composite as in claim 8, wherein said monomer is present in an amount of from 20 to 70 percent by weight of the dental composite.

10. The dental composite as in claim 9, wherein said monomer is present in an amount of from 30 to 50 percent by weight of the dental composite.

## Patentansprüche

1. Dentalkomposit, enthaltend ein photopolymerisierbares und mit Licht spaltbares Harzmonomer, umfassend eine reaktive lichtempfindliche α-Hydroxyalkylphenongruppe und mindestens zwei photopolymerisierbare Gruppen, ausgewählt aus Vinyl-, Vinylether-, Acrylat- und Methacrylatgruppen.

2. Der Dentalkomposit nach Anspruch 1, wobei das α-Hydroxyalkylphenon 4-(2-Hydroxyethoxy)-phenyl-2-hydroxy-2-methyl-2-propanon ist.

3. Der Dentalkomposit nach Anspruch 1, ferner umfassend Ester-, Carbonat-, Harnstoff-, Urethan- oder Etherbindungen sowie Kombinationen daraus.

4. Der Dentalkomposit nach Anspruch 1, mit einem Derivat eines urethanbasierten Monomers, das über einen einstufigen oder einen zweistufigen Prozess erzeugt wurde.

5. Der Dentalkomposit nach Anspruch 4, wobei das urethan-basierte Monomer ausgewählt wird aus der Gruppe, bestehend aus Alkylendiisocyanaten, deren Alkylengruppe 2 bis 18 Kohlenstoffatome aufweist, sowie Arylen- und substituierten Arylen-di- und polyisocyanate.

6. Der Dentalkomposit nach Anspruch 5, wobei die Cyanate ausgewählt werden aus der Gruppe, bestehend aus Ethylendiisocyanat, Propylendiisocyanat, Tetramethylendiisocyanat, Pentamethylendiisocyanat, Hexamethylendiisocyanat (HDI), Hexamethylendiisocyanatbiuret, Hexamethylendiisocyanattrimer(isocyanurat), Octamethylendiisocyanat, Decamethylendiisocyanat, Undecamethylendiisocyanat, Dodecamethylendiisocyanat, Isophorondiisocyanat (IPDI), hydrogeniertes Diphenylmethandiisocyanat (Hi2MDI), Xylylen-1 ,4-diisocyanat (p-XDI), Xylylen-1,3-diisocyanat (m-XDI), m-phenylendiisocyanat, p-phenylendüsocyanat, Toluol-2,6-diisocyanat (2,6-TDI), Toluol-2,4-diisocyanat (2,4-TDI), Mesitylendiisocyanat, Durylendiisocyanat, Benzidendiisocyanat, 1-Methylphenylen-2,4-diisocyanat, Naphtalin-1,4-diisocyanate, 1,2,4-Benzoltrüsocyanat, 4,4'-diisocyanatodiphenylmethan, (MDI), 3,3'-Dimethyl-4,4'diisocyanatodiphenylmethan, 4,4'-Diphenylpropandiisocyanat, Dianisidindiisocyanat, m-Tetramethylenxyloldiisocyanat (TMXDI), sowie geeignete Methycrylate, enthaltend eine Hydroxylgruppe, wie 2-Hydroxyethyl(meth)acrylat, 2-Hydroxypropyl(meth)acrylat, 3-(acryloxyl)-2-hydroxypropyl(meth)acrylat, Diethylenglycolmonohydroxyl(meth)acrylat, Triethylenglycolmonohydroxyl(meth)acrylat, Tetraethylenglycolmonohydroxyl(meth)acrylat, Polyethylenglycolmonohydroxyl(meth)acrylat, sowie Kombinationen daraus.

7. Der Dentalkomposit nach Anspruch 1, das ferner mindestens ein zusätzliches Harz umfasst, ausgewählt aus der Gruppe bestehend aus BisGMA, TEGDMA, UDMA und HEMA.

8. Der Dentalkomposit nach Anspruch 7, wobei das Komposit ein Monomer in einer Menge von 1 bis 99 Gewichtsprozent des Dentalkomposits enthält.

9. Der Dentalkomposit nach Anspruch 8, wobei das Monomer in einer Menge von 20 bis 70 Gewichtsprozent, bezogen auf den Dentalkomposit, vorliegt.

10. Der Dentalkomposit nach Anspruch 9, wobei das Monomer in einer Menge von 30 bis 50 Gewichtsprozent, bezogen auf den Dentalkomposit, vorliegt.

## Revendications

1. Composite dentaire contenant un monomère de résine photopolymérisable et photoclivable comprenant un groupement α-hydroxyalkylphénone photosensible réactif et au moins deux groupes photopolymérisables choisis dans le groupe consistant en des groupes vinyle, éther vinylique, acrylate et méthacrylate.

2. Composite dentaire suivant la revendication 1, dans lequel ladite α-hydroxyalkylphénone est la 4-(2-hydroxy-éthoxy)-phényl-2-hydroxy-2-méthyl-2-propanone.

3. Composite dentaire suivant la revendication 1, comprenant en outre des liaisons ester, carbonate, urée, uréthanne ou éther et leurs associations.

4. Composite dentaire suivant la revendication 1, formé comme dérivé d'un monomère à base d'uréthanne par un procédé en une étape ou en deux étapes.

5. Composite dentaire suivant la revendication 4, dans lequel ledit monomère à base d'uréthanne est choisi dans le groupe consistant en des diisocyanates d'alkylène, dans lesquels ledit groupe alkylène a 2 à 18 atomes de carbone, et des di- et polyisocyanates d'arylène et d'arylène substitué.

6. Composite dentaire suivant la revendication 5, dans lequel lesdits cyanates sont choisis dans le groupe consistant en le diisocyanate d'éthylène, le diisocyanate de propylène, le diisocyanate de tétraméthylène, le diisocyanate de pentaméthylène, le diisocyanate d'hexaméthylène (HDI), le biuret de diisocyanate d'hexaméthylène, le trimère de diisocyanate d'hexaméthylène (isocyanurate), le diisocyanate d'octaméthylène, le diisocyanate de décaméthylène, le diisocyanate d'undécaméthylène, le diisocyanate de dodécaméthylène, le diisocyanate d'isophorone (IPDI), le diisocyanate de diphénylméthane hydrogéné (Hi2MDI), le 1,4-diisocyanate de xylylène (p-XDI), le 1,3-diisocyanate de xylylène (m-XDI), le diisocyanate de m-phénylène, le diisocyanate de p-phénylène, de 2,6-diisocyanate de toluène (2,6-TDI), le 2,4-diisocyanate de toluène (2,4-TDI), le diisocyanate de mésitylène, le diisocyanate de durylène, le diisocyanate de benzidène, le 2,4-diisocyanate de 1-méthylphénylène, le 1,4-diisocyanate de naphtylène, le 1,2,4-triisocyanate de benzène, le 4,4'-diisocyanato-diphénylméthane (MDI), le 3,3'-diméthyl-4,4'-diisocyanato-diphénylméthane, le diisocyanate de 4,4'-diphenyl-propane, le diisocyanate de dianisidine, le diisocyanate m-tétraméthylènexylène (TMXDI) ; en outre, un (méth)acrylate convenable contenant un groupe hydroxyle comprend : le (méth)acrylate de 2-hydroxyéthyle, le (méth)acrylate de 2-hydroxypropoyle, le (méth)acrylate de 3-(acryloxy)-2-hydroxy-propyle, le monohydroxy(méth)acrylate de diéthylèneglycol, le monohydroxy(méth)acrylate de triéthylèneglycol, le mono-hydroxy(méth)acrylate de tétraéthylèneglycol, le monohydroxy-(méth)acrylate de polyéthylèneglycol et leurs associations.

7. Composite dentaire suivant la revendication 1, comprenant en outre au moins une résine supplémentaire choisie dans le groupe consistant en BisGMA, TEGDMA, UDMA et HEMA.

8. Composite dentaire suivant la revendication 7, ledit composite comprenant un monomère en une quantité de 1 à 99 % en poids du composite dentaire.

9. Composite dentaire suivant la revendication 8, dans lequel ledit monomère est présent en une quantité de 20 à 70 % en poids du composite dentaire.

10. Composite dentaire suivant la revendication 9, dans lequel ledit monomère est présent en une quantité de 30 à 50 % en poids du composite dentaire.
